(19) **Europäisches Patentamt / European Patent Office / Office européen des brevets**

(11) **EP 1 485 411 B1**

(12) **EUROPEAN PATENT SPECIFICATION**

(45) Date of publication and mention
of the grant of the patent:
**09.05.2007 Bulletin 2007/19**

(51) Int Cl.:
**C07K 14/705** (2006.01)    **C07K 14/72** (2006.01)
**C07K 1/107** (2006.01)    **G01N 33/68** (2006.01)

(21) Application number: **03708346.6**

(22) Date of filing: **13.03.2003**

(86) International application number:
**PCT/GB2003/001049**

(87) International publication number:
**WO 2003/078464 (25.09.2003 Gazette 2003/39)**

(54) **ARRAYS OF CYTOSOLIC ACCESSORY PROTEINS IMMOBILIZED ON A SURFACE AND PERTINENT METHODS**

ARRAYS ENTHALTEND AUF EINER FESTEN OBERFLÄCHE GEBUNDENE ZYTOSOLISCHE PROTEINE UND BETREFFENDE METHODEN

RESEAUX CONTENANT DES PROTÉINES CYTOSOLIQUES ACCESSOIRES IMMOBILISÉES SUR UNE SURFACE ET PROCEDES ASSOCIES

(84) Designated Contracting States:
**AT BE BG CH CY CZ DE DK EE ES FI FR GB GR HU IE IT LI LU MC NL PT RO SE SI SK TR**

(30) Priority: **13.03.2002 GB 0205910**

(43) Date of publication of application:
**15.12.2004 Bulletin 2004/51**

(73) Proprietor: **SENSE PROTEOMIC LIMITED**
**Maidenhead, Berkshire SL6 7RJ (GB)**

(72) Inventors:
- **KOZLOWSKI, Roland**
  **Wootton, Woodstock (GB)**
- **BLACKBURN, Jonathan, Michael**
  **7535 Belleville,**
  **Cape Town (ZA)**
- **DAVIES, Andrew**
  **Bristol BS6 7JW (GB)**
- **GODBER, Benjamin, Leslie J.**
  **Cambridge CB3 6AP (GB)**

- **HART, Darren, James,**
  **c/o Sense Proteomic limited**
  **Nedging Tye,**
  **Ipswich IP7 7HQ (GB)**

(74) Representative: **Aston, Heidi Frances et al**
**Mintz, Levin, Cohn, Ferris, Glovsky**
**& Popeo Intellectual Property LLP**
**The Rectory**
**9 Ironmonger Lane**
**London, EC2V 8EY (GB)**

(56) References cited:
**WO-A-00/54046**    **WO-A-01/57198**

- **H ZHU ET AL.: "Analysis of yeast protein kinases using protein chips" NATURE GENETICS., vol. 26, no. 3, November 2000 (2000-11), pages 283-289, XP002258127 NATURE AMERICA, NEW YORK., US ISSN: 1061-4036 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

SEQUENCE LISTING

<110> Sense proteomic Limited
Blackburn, Jonathan M
Kozlowski, Roland Z
Davies, Andrew
Godber, Ben
Hart, Darren

<120> Arrays and Methods

<130> P33676wo

<140> PCT/GB2003/001049
<141> 2003-03-13

<150> GB 0205910.3
<151> 2002-03-13

<160> 6

<170> PatentIn version 3.2

<210> 1
<211> 334
<212> PRT
<213> Homo sapiens

<400> 1

```
Gly Thr Gly Met Lys Tyr Arg Asn Leu Gly Lys Ser Gly Leu Arg Val
1               5               10              15

Ser Cys Leu Gly Leu Gly Thr Trp Val Thr Phe Gly Gly Gln Ile Ser
            20              25              30

Asp Glu Val Ala Glu Arg Leu Met Thr Ile Ala Tyr Glu Ser Gly Val
            35              40              45

Asn Leu Phe Asp Thr Ala Glu Val Tyr Ala Ala Gly Lys Ala Glu Val
        50              55              60

Ile Leu Gly Ser Ile Ile Lys Lys Lys Gly Trp Arg Arg Ser Ser Leu
65              70              75              80

Val Ile Thr Thr Lys Leu Tyr Trp Gly Gly Lys Ala Glu Thr Glu Arg
                85              90              95

Gly Leu Ser Arg Lys His Ile Ile Glu Gly Leu Lys Gly Ser Leu Gln
            100             105             110

Arg Leu Gln Leu Glu Tyr Val Asp Val Val Phe Ala Asn Arg Pro Asp
        115             120             125

Ser Asn Thr Pro Met Glu Glu Ile Val Arg Ala Met Thr His Val Ile
    130             135             140

Asn Gln Gly Met Ala Met Tyr Trp Gly Thr Ser Arg Trp Ser Ala Met
145             150             155             160
```

```
Glu Ile Met Glu Ala Tyr Ser Val Ala Arg Gln Phe Asn Met Ile Pro
              165             170              175

Pro Val Cys Glu Gln Ala Glu Tyr His Leu Phe Gln Arg Glu Lys Val
              180             185             190

Glu Val Gln Leu Pro Glu Leu Tyr His Lys Ile Gly Val Gly Ala Met
              195             200             205

Thr Trp Ser Pro Leu Ala Cys Gly Ile Ile Ser Gly Lys Tyr Gly Asn
    210             215             220

Gly Val Pro Glu Ser Ser Arg Ala Ser Leu Lys Cys Tyr Gln Trp Leu
225             230             235             240

Lys Glu Arg Ile Val Ser Glu Glu Gly Arg Lys Gln Gln Asn Lys Leu
              245             250             255

Lys Asp Leu Ser Pro Ile Ala Glu Arg Leu Gly Cys Thr Leu Pro Gln
              260             265             270

Leu Ala Val Ala Trp Cys Leu Arg Asn Glu Gly Val Ser Ser Val Leu
              275             280             285

Leu Gly Ser Ser Thr Pro Glu Gln Leu Ile Glu Asn Leu Gly Ala Ile
    290             295             300

Gln Val Leu Pro Lys Met Thr Ser His Val Val Asn Glu Ile Asp Asn
305             310             315             320

Ile Leu Arg Asn Lys Pro Tyr Ser Lys Lys Asp Tyr Arg Ser
              325             330
```

<210> 2
<211> 333
<212> PRT
<213> Homo sapiens

<400> 2

```
Gly Leu Gln Phe Tyr Arg Asn Leu Gly Lys Ser Gly Leu Arg Val Ser
1               5               10              15

Cys Leu Gly Leu Gly Thr Trp Val Thr Phe Gly Gly Gln Ile Thr Asp
              20              25              30

Glu Met Ala Glu Gln Leu Met Thr Leu Ala Tyr Asp Asn Gly Ile Asn
    35              40              45

Leu Phe Asp Thr Ala Glu Val Tyr Ala Ala Gly Lys Ala Glu Val Val
    50              55              60
```

Leu Gly Asn Ile Ile Lys Lys Lys Gly Trp Arg Arg Ser Ser Leu Val
65                  70              75                      80

Ile Thr Thr Lys Ile Phe Trp Gly Gly Lys Ala Glu Thr Glu Arg Gly
                85              90                  95

Leu Ser Arg Lys His Ile Ile Glu Gly Leu Lys Ala Ser Leu Glu Arg
            100             105             110

Leu Gln Leu Glu Tyr Val Asp Val Val Phe Ala Asn Arg Pro Asp Pro
        115             120             125

Asn Thr Pro Met Glu Glu Thr Val Arg Ala Met Thr His Val Ile Asn
    130             135             140

Gln Gly Met Ala Met Tyr Trp Gly Thr Ser Arg Trp Ser Ser Met Glu
145             150             155             160

Ile Met Glu Ala Tyr Ser Val Ala Arg Gln Phe Asn Leu Thr Pro Pro
            165             170             175

Ile Cys Glu Gln Ala Glu Tyr His Met Phe Gln Arg Glu Lys Val Glu
            180             185             190

Val Gln Leu Pro Glu Leu Phe His Lys Ile Gly Val Gly Ala Met Thr
        195             200             205

Trp Ser Pro Leu Ala Cys Gly Ile Val Ser Gly Lys Tyr Asp Ser Gly
    210             215             220

Ile Pro Pro Tyr Ser Arg Ala Ser Leu Lys Gly Tyr Gln Trp Leu Lys
225             230             235             240

Asp Lys Ile Leu Ser Glu Glu Gly Arg Arg Gln Gln Ala Lys Leu Lys
            245             250             255

Glu Leu Gln Ala Ile Ala Glu Arg Leu Gly Cys Thr Leu Pro Gln Leu
        260             265             270

Ala Ile Ala Trp Cys Leu Arg Asn Glu Gly Val Ser Ser Val Leu Leu
        275             280             285

Gly Ala Ser Asn Ala Asp Gln Leu Met Glu Asn Ile Gly Ala Ile Gln
    290             295             300

Val Leu Pro Lys Leu Ser Ser Ser Ile Ile His Glu Ile Asp Ser Ile
305             310             315             320

Leu Gly Asn Lys Pro Tyr Ser Lys Lys Asp Tyr Arg Ser
            325             330

<210> 3
<211> 330
<212> PRT
<213> Homo sapiens

<400> 3

Gly Thr Gly Met Lys Tyr Arg Asn Leu Gly Lys Ser Gly Leu Arg Val
1               5                   10                  15

Ser Cys Leu Gly Leu Gly Thr Trp Val Thr Phe Gly Ser Gln Ile Ser
            20                  25                  30

Asp Glu Thr Ala Glu Asp Val Leu Thr Val Ala Tyr Glu His Gly Val
            35                  40                  45

Asn Leu Phe Asp Thr Ala Glu Val Tyr Ala Ala Gly Lys Ala Glu Arg
        50                  55                  60

Thr Leu Gly Asn Ile Leu Lys Ser Lys Gly Trp Arg Arg Ser Ser Tyr
65                  70                  75                  80

Val Ile Thr Thr Lys Ile Phe Trp Gly Gly Gln Ala Glu Thr Glu Arg
                85                  90                  95

Gly Leu Ser Arg Lys His Ile Ile Glu Gly Leu Arg Gly Ser Leu Glu
            100                 105                 110

Arg Leu Gln Leu Gly Tyr Val Asp Ile Val Phe Ala Asn Arg Ser Asp
        115                 120                 125

Pro Asn Cys Pro Met Glu Glu Ile Val Arg Ala Met Thr Tyr Val Ile
    130                 135                 140

Asn Gln Gly Leu Ala Leu Tyr Trp Gly Thr Ser Arg Trp Gly Ala Ala
145                 150                 155                 160

Glu Ile Met Glu Ala Tyr Ser Met Ala Arg Gln Phe Asn Leu Ile Pro
            165                 170                 175

Pro Val Cys Glu Gln Ala Glu His His Leu Phe Gln Arg Glu Lys Val
            180                 185                 190

Glu Met Gln Leu Pro Glu Leu Tyr His Lys Ile Gly Val Gly Ser Val
            195                 200                 205

Thr Trp Tyr Pro Leu Ala Cys Gly Leu Ile Thr Ser Lys Tyr Asp Gly
    210                 215                 220

Arg Val Pro Asp Thr Cys Arg Ala Ser Ile Lys Gly Tyr Gln Trp Leu
225                 230                 235                 240

```
Lys Asp Lys Val Gln Ser Glu Asp Gly Lys Lys Gln Gln Ala Lys Val
                245                 250                 255

Met Asp Leu Leu Pro Val Ala His Gln Leu Gly Cys Thr Val Ala Gln
                260                 265                 270

Leu Ala Ile Ala Trp Cys Leu Arg Ser Glu Gly Val Ser Ser Val Leu
                275                 280                 285

Leu Gly Val Ser Ser Ala Glu Gln Leu Ile Glu His Leu Gly Ala Leu
        290                 295                 300

Gln Val Leu Ser Gln Leu Thr Pro Gln Thr Val Met Glu Ile Asp Gly
305                 310                 315                 320

Leu Leu Gly Asn Lys Pro His Ser Lys Lys
                325                 330
```

<210> 4
<211> 334
<212> PRT
<213> Rattus sp.

<400> 4

Gly Thr Gly Met Lys Tyr Arg Asn Leu Gly Lys Ser Gly Leu Arg Val
1               5                   10              15

Ser Cys Leu Gly Leu Gly Thr Trp Val Thr Phe Gly Gly Gln Ile Ser
            20              25              30

Asp Glu Val Ala Glu Arg Leu Met Thr Ile Ala Tyr Glu Ser Gly Val
        35              40              45

Asn Leu Phe Asp Thr Ala Glu Val Tyr Ala Ala Gly Lys Ala Glu Val
    50              55              60

Ile Leu Gly Ser Ile Ile Lys Lys Lys Gly Trp Arg Arg Ser Ser Leu
65              70              75              80

Val Ile Thr Thr Lys Leu Tyr Trp Gly Gly Lys Ala Glu Thr Glu Arg
            85              90              95

Gly Leu Ser Arg Lys His Ile Ile Glu Gly Leu Lys Gly Ser Leu Gln
            100             105             110

Arg Leu Gln Leu Glu Tyr Val Asp Val Val Phe Ala Asn Arg Pro Asp
        115             120             125

Ser Asn Thr Pro Met Glu Glu Ile Val Arg Ala Met Thr His Val Ile
    130             135             140

Asn Gln Gly Met Ala Met Tyr Trp Gly Thr Ser Arg Trp Ser Ala Met

|  | 145 | | | | | 150 | | | | | 155 | | | | | 160 |

```
              145                      150                      155                      160

     Glu Ile Met Glu Ala Tyr Ser Val Ala Arg Gln Phe Asn Met Ile Pro
                     165             170                 175

     Pro Val Cys Glu Gln Ala Glu Tyr His Leu Phe Gln Arg Glu Lys Val
                 180             185                 190

     Glu Val Gln Leu Pro Glu Leu Tyr His Lys Ile Gly Val Gly Ala Met
                 195             200                 205

     Thr Trp Ser Pro Leu Ala Cys Gly Ile Ile Ser Gly Lys Tyr Gly Asn
         210             215             220

     Gly Val Pro Glu Ser Ser Arg Ala Ser Leu Lys Cys Tyr Gln Trp Leu
     225             230             235                 240

     Lys Glu Arg Ile Val Ser Glu Glu Gly Arg Lys Gln Gln Asn Lys Leu
                 245             250                 255

     Lys Asp Leu Ser Pro Ile Ala Glu Arg Leu Gly Cys Thr Leu Pro Gln
                 260             265                 270

     Leu Ala Val Ala Trp Cys Leu Arg Asn Glu Gly Val Ser Ser Val Leu
                 275             280                 285

     Leu Gly Ser Ser Thr Pro Glu Gln Leu Ile Glu Asn Leu Gly Ala Ile
         290             295             300

     Gln Val Leu Pro Lys Met Thr Ser His Val Val Asn Glu Ile Asp Asn
     305             310             315                 320

     Ile Leu Arg Asn Lys Pro Tyr Ser Lys Lys Asp Tyr Arg Ser
                 325             330
```

<210> 5
<211> 333
<212> PRT
<213> Rattus sp.

<400> 5

Gly Leu Gln Phe Tyr Arg Asn Leu Gly Lys Ser Gly Leu Arg Val Ser
1                   5                   10                  15

Cys Leu Gly Leu Gly Thr Trp Val Thr Phe Gly Gly Gln Ile Thr Asp
                20                  25                  30

Glu Met Ala Glu His Leu Met Thr Leu Ala Tyr Asp Asn Gly Ile Asn
            35                  40                  45

Leu Phe Asp Thr Ala Glu Val Tyr Ala Ala Gly Lys Ala Glu Val Val
        50                  55                  60

```
Leu Gly Asn Ile Ile Lys Lys Lys Gly Trp Arg Arg Ser Ser Leu Val
65              70              75              80

Ile Thr Thr Lys Ile Phe Trp Gly Gly Lys Ala Glu Thr Glu Arg Gly
              85              90              95

Leu Ser Arg Lys His Ile Ile Glu Gly Leu Lys Ala Ser Leu Glu Arg
            100             105             110

Leu Gln Leu Glu Tyr Val Asp Val Val Phe Ala Asn Arg Pro Asp Pro
        115             120             125

Asn Thr Pro Met Glu Glu Thr Val Arg Ala Met Thr His Val Ile Asn
    130             135             140

Gln Gly Met Ala Met Tyr Trp Gly Thr Ser Arg Trp Ser Ser Met Glu
145             150             155             160

Ile Met Glu Ala Tyr Ser Val Ala Arg Gln Phe Asn Leu Ile Pro Pro
            165             170             175

Ile Cys Glu Gln Ala Glu Tyr His Met Phe Gln Arg Glu Lys Val Glu
        180             185             190

Val Gln Leu Pro Glu Leu Phe His Lys Ile Gly Val Gly Ala Met Thr
        195             200             205

Trp Ser Pro Leu Ala Cys Gly Ile Val Ser Gly Lys Tyr Asp Ser Gly
    210             215             220

Ile Pro Pro Tyr Ser Arg Ala Ser Leu Lys Gly Tyr Gln Trp Leu Lys
225             230             235             240

Asp Lys Ile Leu Ser Glu Glu Gly Arg Arg Gln Gln Ala Lys Leu Lys
            245             250             255

Glu Leu Gln Ala Ile Ala Glu Arg Leu Gly Cys Thr Leu Pro Gln Leu
        260             265             270

Ala Ile Ala Trp Cys Leu Arg Asn Glu Gly Val Ser Ser Val Leu Leu
    275             280             285

Gly Ala Ser Asn Ala Glu Gln Leu Met Glu Asn Ile Gly Ala Ile Gln
290             295             300

Val Leu Pro Lys Leu Ser Ser Ser Ile Val His Glu Ile Asp Ser Ile
305             310             315             320

Leu Gly Asn Lys Pro Tyr Ser Lys Lys Asp Tyr Arg Ser
            325             330
```

<210> 6
<211> 330
<212> PRT
<213> Rattus sp.

<400> 6

```
Gly Thr Gly Met Lys Tyr Arg Asn Leu Gly Lys Ser Gly Leu Arg Val
1               5               10              15

Ser Cys Leu Gly Leu Gly Thr Trp Val Thr Phe Gly Ser Gln Ile Ser
            20              25              30

Asp Glu Thr Ala Glu Asp Leu Leu Thr Val Ala Tyr Glu His Gly Val
        35              40              45

Asn Leu Phe Asp Thr Ala Glu Val Tyr Ala Ala Gly Lys Ala Glu Arg
        50              55              60

Thr Leu Gly Asn Ile Leu Lys Ser Lys Gly Trp Arg Arg Ser Ser Tyr
65              70              75              80

Val Ile Thr Thr Lys Ile Phe Trp Gly Gly Gln Ala Glu Thr Glu Arg
                85              90              95

Gly Leu Ser Arg Lys His Ile Ile Glu Gly Leu Gln Gly Ser Leu Asp
            100             105             110

Arg Leu Gln Leu Glu Tyr Val Asp Ile Val Phe Ala Asn Arg Ser Asp
            115             120             125

Pro Ser Ser Pro Met Glu Glu Ile Val Arg Ala Met Thr Tyr Val Ile
    130             135             140

Asn Gln Gly Leu Ala Leu Tyr Trp Gly Thr Ser Arg Trp Ser Ala Ala
145             150             155             160

Glu Ile Met Glu Ala Tyr Ser Met Ala Arg Gln Phe Asn Leu Ile Pro
            165             170             175

Pro Val Cys Glu Gln Ala Glu Asn His Phe Phe Gln Arg Glu Lys Val
            180             185             190

Glu Met Gln Leu Pro Glu Leu Tyr His Lys Ile Gly Val Gly Ser Val
            195             200             205

Thr Trp Ser Pro Leu Ala Cys Ser Leu Ile Thr Ser Lys Tyr Asp Gly
    210             215             220

Gln Val Pro Asp Ala Cys Lys Ala Thr Val Lys Gly Tyr Gln Trp Leu
225             230             235             240
```

```
Lys Glu Lys Val Gln Ser Glu Asp Gly Lys Lys Gln Gln Ala Arg Val
            245             250             255

Thr Asp Leu Leu Pro Ile Ala His Gln Leu Gly Cys Thr Val Ala Gln
            260             265             270

Leu Ala Ile Ala Trp Cys Leu Arg Ser Glu Gly Val Ser Ser Val Leu
            275             280             285

Leu Gly Val Ser Ser Ala Glu Gln Leu Met Glu His Leu Gly Ser Leu
    290             295             300

Gln Val Leu Gly Gln Leu Thr Pro Gln Thr Val Met Glu Ile Asp Ala
305             310             315             320

Leu Leu Gly Asn Lys Ser His Ser Lys Lys
            325             330
```

**Description**

The present invention relates to certain protein arrays and their use in screening methods.

A multitude of cellular signalling and homeostatic processes are mediated by transmembrane proteins such as ion channels, receptors and transporters. All of these protein classes interconnect with signalling, regulatory, scaffolding and adapter proteins through protein-protein interactions involving cytoplasmic domains.

Ion channels, membrane bound receptors such as G-protein-coupled receptors and many cell surface transporters are comprised of protein subunit complexes, consisting of homomeric or heteromeric assemblies of subunits (termed herein as accessory proteins) on the cytoplasmic face of the membrane. The ability of these protein groups to form complexes in a combinatorial manner provides a mechanism for tissue specific expression of transmembrane protein complexes with the requisite biophysical properties or modulator sensitivity. Increasingly, the determinants of subunit interactions are being located to defined domains within these subunit structures. Understanding the interactions of these domains will aid elucidation of membrane protein assembly in native systems and will identify modulatory proteins important for protein function within a cell. Further, perceived wisdom within these research fields recognises that perturbing the interaction between the accessory cytoplasmic subunits and the membrane bound portion of a transmembrane protein, once it is identified, could represent a highly tissue selective and therefore specific way of regulating the activity of the membrane bound molecule. Exogenous inhibitors of accessory protein interactions and hence subunit or modulatory protein assembly, may therefore provide novel, tissue-specific therapeutic targets.

Voltage gated potassium ($K_V$) channels and calcium ($Ca^{2+}$ channels are recognised as important therapeutic targets in many disorders including those of the CNS, heart, lungs and bladder. Both channel types have a principal pore-forming, voltage-sensing $\alpha$-subunit. Kv channels require assembly of four similar $\alpha$-subunits, each containing six transmembrane segments, whereas calcium channels have a single $\alpha$-subunit with 24 transmembrane segments, packaged into four 'pseudosubunits' of six transmembrane segments [1]. Both Kv and $Ca^{2+}$ channels co-assemble with structurally similar cytoplasmic $\beta$-subunits via well-defined domains within the $\alpha$-subunit. These can affect channel gating to various extents and can dramatically alter surface expression efficiency of $\alpha$-subunits through mechanisms that may resemble or mimic the interaction of membrane proteins with authentic molecular chaperones [2]. To date, there have been six $\beta$-subunits identified for each of the two channel types. The ability of the $Ca^{2+}$ channel $\beta$-subunits to assemble with the six $\alpha$-subunits has been determined [3] but the interactions of the Kv channel $\beta$-subunits with the twenty or so $\alpha$-subunits is less well established and it is not yet known in detail which combinations of $\beta$- and $\alpha$ subunits are truly biologically relevant since the methods of analysis currently available are relatively crude (see below).

Receptors, in particular G-protein coupled receptors, are recognised as an extremely important group of cell surface proteins and they perform a wide variety of functions in many systems [4]. These 7-12 transmembrane proteins are grouped into three families, A, B and C, within which are many subtypes. Each GPCR interacts with at least one G-protein at its cytoplasmic face although the nature and identity of these interactions is poorly understood and is currently impossible to predict from bioinformatics. G-proteins, as well as complexing with GPCR's also interact with K+ and $Ca^{2+}$ channel subunits in a specific manner with striking results [5].

Proteins at the cell surface that allow entry of bulky molecules represent a third class of membrane proteins, transporters, that complex with multiple cytosolic protein subunits in order to function, but here again the exact nature and identity of these interactions is poorly characterised at present due to limitations in current analysis methodologies.

New methodologies which enable the highly parallel *in vitro* characterisation, including determination of equilibrium and kinetic binding parameters, of protein subunit binding interactions between various putative subunit partners will be tremendously useful in this field, particularly if they also allow assessment of the ability of potentially therapeutic compounds to modulate such interactionsCurrent methods for studying these interactions rely on cumbersome cell-based techniques such as electrophysiological analysis, or low throughput *in vitro* binding systems such as pull-down/immuno-precipitation assays. By contrast, the present invention describes an array-based method whereby the *in vitro* binding of soluble, functional domains of membrane-bound proteins, such as ion channels, cell surface receptors and transporters to arrays of immobilised cytoplasmic accessory proteins can be characterised in detail. The invention allows binding affinity and specificity of the protein-protein interaction to be determined and also allows the actions of inhibitors/competitors of the interaction to be explored in a highly parallel system.

The yeast two-hybrid system has been used to detect interactions between, for example, ion channel protein domains. This approach has been reasonably successful in identifying binding domains and in determining the effect of amino acid substitutions on the interaction. However, this method cannot provide quantitative information about binding affinities and cannot address the potential of exogenous substances to modulate the interactions. Alternatively, cell-based systems, for example whole-cell electrophysiology following subunit expression in *Xenopus* oocytes or mammalian cell lines, have been used to study ion channel assembly. This approach is technically difficult, inherently time-consuming and provides limited information about the actual binding events due to the measurement of downstream effects, ie whole-cell currents.

Biochemical methods have been used to isolate individual components of ion channel assemblies in native and recombinant systems. Coimmunoprecipitation experiments and affinity purification of channel subunits have been used to identify components of ion channel complexes. However, neither method provides detailed information about binding affinities or is suitable for screening potential modulators of subunit interactions. In the case of $Ca^{2+}$ channels, fragments of one particular $\alpha$-subunit have been immobilised on agarose beads as fusions to GST and the binding of *in vitro* translated $\beta$-subunits was used to more closely define the specific region of the $\alpha$-subunit involved in the interaction [6]. Detailed information on binding affinities were not available in this study and it is not clear that the alpha subunit was correctly folded in this study.

Protein overlay binding examines binding interactions directly between a soluble peptide/protein and a target protein immobilised on a membrane support [7]. During this procedure proteins are initially dissociated and separated by SDS-PAGE and transferred to a nitrocellulose filter as for Western blotting. The denaturing step implicit in this method however suggests that the proteins assayed in this technique cannot be functional (i.e. folded) and therefore biologically active. Attempts can be made to renature the protein before binding of a soluble, labelled protein is investigated however the success rate here is poor.

Identification of native interactions between membrane proteins and accessory proteins, and the binding domains involved, have been investigated using various cell-based methods. For example, the native combinations of $\alpha$ and $\beta$ Kv channel subtypes within a complex have been probed using co-purification with antibodies or subtype-specific toxins [9,10]. Yeast two-hybrid experiments have been widely used to identify protein-protein interaction domains and, for example, were used to identify the region of the N-terminal T1 domain of Kv channels that interacts with the cytoplasmic $\beta$ subunits [11]. The ability of proteins to interact in a model cell has been investigated using recombinant expression techniques. Examples include co-expression of voltage-gated ion channels and functional characterisation using electrophysiology [3,11,12], and the expression of chimeric and mutant GPCRs [reviewed in 13,14]. The perceived wisdom derived from such experiments is that the $\alpha$ and $\beta$ subunits form a stable complex in the ER which does not subsequently dissociate.

Binding interactions between domains of membrane proteins and the domains of other subunits or their cytoplasmic partners, following separate expression, have been studied using pull-down assays on a one-by-one basis. The tetramerisation domains of $K^+$ channel transmembrane subunits have been examined in this way [15]. Intracellular domains of $Ca^{2+}$ channels binding to $\beta$ subunits [6] or SNARE proteins [16], the 5-HT$_{2A}$ GPCR binding to arrestins [17] and the CFTR transporter binding to the adapter protein AP-2 [18] have been examined by immobilising the membrane protein domain on beads. Alternatively, the cytoplasmic Homer protein has been immobilised on beads to show the interaction with metabotropic glutamate receptors expressed in a cell lysate [19]. Cross-linking experiments have been performed between CFTR and AP-2 in microtiter plates [18], but in this case proteins were not immobilised. Protein-overlay experiments allow a number of binding interactions to be identified simultaneously. This technique was used to show binding of labelled Drosophila INAD to immobilised TRP $Ca^{2+}$ channels [20]. This technique allows a number of proteins to be fractionated in different lanes of an SDS-PAGE gel and then immobilised on a nitrocellulose filter to be probed for binding.

The nature of the techniques described above means that the protein analysis usually either involves co-expression of the potential interacting partner, or involves denaturation of one or more of the potential partners, prior to the binding assay. Invariably they are low throughput and do not yield detailed information on binding specificity or affinity and are not generally compatible with small molecule inhibitor studies. To overcome such problems, the Inventors have devised an array of separate, and importantly, functional, accessory proteins that were not originally co-expressed with the membrane protein components. Such an array allows, for the first time, the quantitative determination of interactions in a highly parallel manner.

Thus in a first aspect the invention provides an array comprising a surface having attached thereto at least one cytosolic accessory protein free of it's membrane protein components or other subunits with which it is normally complexed. Preferably, the cytosolic accessory proteins are cytosolic accessory proteins of membrane proteins which are members of a family of homologous membrane proteins. In one embodiment, the family of homologous membrane proteins is selected from the group consisting of ion-channels, G protein coupled receptors and transmembrane transporter proteins.

The accessory proteins on the array can be members of a family of homologous accessory proteins, for example ion-channel subunits (for example, $\beta$-subunits), receptor interacting proteins (for example, G proteins, arrestins and G protein receptor kinases) or accessory proteins for transporters (for example transporter protein interacting proteins). Particularly envisaged are arrays wherein the accessory proteins are K+-channel $\beta$-subunits, Ca2+-channel $\beta$-subunits, G protein subtypes, for example, G$\alpha$, G$\beta$/$\gamma$ or accessory proteins for transporters. Examples include Kv $\beta$-subunits channel e.g. $\beta$1.1, 1.2, 1.3, 2.1, 2.2, 3.1, 3.2, 4, Calcium channel $\beta$-subunits e.g $\beta$1a, $\beta$1b, $\beta$1c, $\beta$2a, $\beta$2b, $\beta$2c, $\beta$3a, $\beta$3b, $\beta$4, G protein families e.g. G$_s$ family ($\alpha_s$ and $\alpha_{olf}$), Gt family, G$_i$ family ($\alpha_o$, $\alpha_{i1}$-$\alpha_{i13}$, $\alpha_z$), G$_{i-0}$ family, G$_{q-11}$ family ($\alpha_q$, $\alpha_{11}$, $\alpha_{14}$, $\alpha_{15}$, $\alpha_{16}$, $\alpha_{12}$, $\alpha_{13}$) and G$_{\alpha\text{-sensory}}$ family ($\alpha_{t\text{-rod}}$, $\alpha_{gust}$) and $\beta\gamma$ family ($\gamma$t,$\gamma$1,$\gamma$2,$\gamma$3 etc.), or accessory proteins to transporter proteins (for example seratonin and glycine transporter proteins).

The number of proteins attached to the arrays of the invention will be determined, at least to a certain extent, by the number of proteins that occur naturally or that are of sufficient experimental, commercial or clinical interest. An array carrying one or two proteins would be of use to the investigator. However in practice and in order to take advantage of the suitability of such arrays for high throughput assays, it is envisaged that 1 to 10000, 1 to 1000, 1 to 500, 1 to 400, 1 to 300, 1 to 200, 1 to 100, 1 to 75, 1 to 50, 1 to 25, 1 to 10 or 1 to 5 such proteins are present on an array.

In a second aspect, the invention provides a method for determining which cytosolic accessory proteins interact with a given membrane protein or vice versa, said method comprising the steps of:

(i) providing an array of candidate cytosolic accessory proteins free of their membrane protein components or other subunits with which they are normally complexed from one or more cytosolic accessory protein families of interest;
(ii) contacting the array with cytosolic fragments of said membrane protein and/or cytosolic fragments of other related membrane protein family members;and
(iii) detecting and identifying the interacting partners.

In a third aspect the invention provides a method for screening compounds or peptides or proteins for the ability to interact selectively with a cytosolic accessory protein, said method comprising the steps of:

(i) providing an array of cytosolic accessory proteins free of their membrane protein components or other subunits with which they are normally complexed from one or more cytosolic protein families of interest;
(ii) contacting the array with compounds or peptides or proteins;and
(iii) identifying the interacting partners.

This method optionally comprises the additional step (iv) of quantitating the interaction of the interacting partners.

Also provided is a method for screening compounds or peptides or proteins for the ability to selectively modulate the interaction between a cytosolic accessory protein and a membrane protein, said method comprising the steps of

(i) providing an array of cytosolic accessory proteins free of their membrane protein components or other subunits with which they are normally complexed from one or more protein families of interest;
(ii) contacting the array with compounds or peptides or proteins and with one or more membrane proteins or cytosolic fragments thereof of interest, either simultaneously or in sequence; and
(iii) determining whether said interaction is modulated by the presence of said compounds or peptides or proteins.

This method optionally comprises the additional step (iv) of quantitating the degree of modulation of the interaction.

The invention also provides the use of an array of cytosolic accessory proteins of the invention to measure the relative catalytic activity of different members of a family of accessory proteins.

The invention also provides the use of an array of cytosolic accessory proteins of the invention as an affinity surface on which to select antibodies from a library of phenotype-genotype-linked antibodies (e.g. phage displayed antibodies).

The invention also provides the use of an array of cytosolic accessory proteins of the invention for determining the effect of post-translational modifications on the interactions of accessory proteins with membrane proteins and/or the properties of said membrane proteins or accessory proteins.

Subunit arrays according to the invention can comprise tagged protein constructs that are each expressed and immobilised in a functional manner in a spatially defined format. The tagged protein constructs can be drawn from groups of accessory protein subunits such as the cytoplasmic auxiliary β-subunits of voltage-gated $K^+$ or $Ca^{2+}$ ion-channels, G-proteins, or globular accessory proteins that interact with bulk transporters. Binding of a labelled probe, which can be another channel subunit, associating protein, interaction domain, peptide or other potential ligand can be investigated using technically simple protocols in a high throughput manner. Binding of complex mixtures or labelled or unlabelled proteins from recombinant systems or cell lysates can also be examined. An array of identical subunits is suited to screening large numbers of potential binding partners and obtaining detailed binding information. Alternatively, an array of different proteins, representing subunits of different subtypes and species is most suited to examining the specificity of ligand binding.

Such arrays can be used to study ion channels, including the ligand-gated ion channel class of receptors, which exist as multi-subunit complexes, consisting of channel-forming subunits, modulatory proteins and proteins ,involved in subcellular location of the channel. Interactions between channel subunits may be mediated by defined intracellular domains. Voltage gated $K^+$ and $Ca^{2+}$ channels co-assemble with structurally similar cytoplasmic β-subunits via well-defined domains within the α-subunit [7,21,22]. These can affect channel gating to various extents and can dramatically alter surface expression efficiency of α-subunits through mechanisms that may resemble or mimic the interaction of membrane proteins with authentic molecular chaperones [2].

Another application of the arrays of the invention is in the study of receptors, for example, G-protein-coupled receptors (GPCRs). These constitute the largest family of cell surface molecules involved in signal transmission, with over 1000 heptahelical proteins identified in the human genome. GPCRs transduce extracellular signals by activating a subset of trimeric G-proteins. Binding sites can be determined for specific GPCR-G-protein partners, such as rhodopsin and the retinal G-protein transducin [23]. However, the binding determinants of the GPCRs do not form discrete domains and it is difficult to predict the subset of G-proteins a receptor will bind to [24]. An array based method for determining such interactions with the large number of orphan GPCRs currently under investigation would be highly advantageous. The effects of GPCRs are modulated for example, by interactions and binding with G-protein-coupled receptor kinases (GRKs) and arrestins, which lead to receptor desensitisation and internalisation [25]. It has been demonstrated that the interaction with arrestin is robust enough to be detected by coimmunoprecipitation [17] and can be inhibited by synthetic peptides [26].
As such, it is a potential target for investigation using an *in vitro* binding method.

A further application of the arrays of the invention is in the study of transmembrane transporter proteins. These proteins control the selective uptake of nutrients and export of metabolic products, regulate the balance of ions and solutes between the exterior and interior of the cell and modulate synaptic transmission by the removal of neurotransmitters from the synaptic cleft. A major regulatory mechanism for these proteins involves translocation to and from cell membranes. However, evidence is emerging that regulation by protein-protein interaction with transporters located in the cell membrane also occurs. e.g. Phosphorylation-dependent *in vitro* binding between the membrane bound *E. coli* transporter enzyme $IICB^{Glc}$ and the global repressor Mlc has indicated a mechanism of transcriptional control [27]. Binding of a C-terminal domain of the glucose transporter GLUT-1 to a transmembrane regulatory protein, stomatin, has also been demonstrated by affinity column chromatography [28].

The term "array" as defined herein refers to a spatially defined arrangement of one or more protein moieties in a pattern on a surface. Preferably the protein moieties are attached to the surface either directly or indirectly. The attachment can be non-specific (e.g. by physical absorption onto the surface or by formation of a non-specific covalent interaction). In a preferred embodiment the protein moieties are attached to the surface through a common marker moiety linked to each protein, for example as described in WO 01/57198.

Thus, for example, each position in the pattern can contain one or more copies of:

a) a sample of a single protein type (in the form of a monomer, dimer, trimer, tetramer or higher multimer);

b) a sample of a single protein type bound to an interacting molecule (e.g. DNA, antibody, other protein);

c) a sample of a single protein type bound to a synthetic molecule (e.g. peptide, chemical compound); or

d) A heteromeric mixture of 2 or more proteins from a given accessory protein family.

The surface which supports the array can be coated/derivatised by chemical treatment, for instance. Examples of suitable surfaces include glass slides, polypropylene or polystyrene, silica, gold or metal support or membranes made of, for example, nitrocellulose, PVDF, nylon or phosphocellulose. The format of the array can be that of a microwell plate or a microarray.

The use of a protein array format according to the invention has many advantages. For example, an array of components of a membrane protein assembly; such as auxiliary subunits, associated proteins and channel subunit domains, permits interactions with binding partners in the solution phase to be examined. Binding parameters can be accurately determined and the effects of modulators or inhibitors of binding can be studied in a highly parallel manner.

A number of recent publications [for example reference 8] have shown that arrays of functional proteins allow the individual members of an array to be screened simultaneously under identical conditions. This allows highly parallel and rapid experiments compared with other techniques, leading to directly comparable results across many proteins. These qualities set protein arrays apart from other assays for protein function, such as Y2H and immunoprecipitations/pull-downs, where the cellular compartmentalisation that is implicit in these other methods effectively divides each protein collection into individual proteins and thus individual assays.

Protein arrays according to the invention can preserve the functional activity of proteins when they are specifically immobilised, allowing biological activity to be directly measured. In addition they provide a direct method for determining the effect of a potential inhibitor entities on on the interactions observed which is not possible in a comparable manner using immunoprecipitations and pull-down assays.

Protein arrays according to the invention can be used to quantitate binding constants ($K_d$) for observed interactions and also allow the concentration of a compound required to inhibit a given interaction to be measured through determination of $IC_{50}$ values.

Results obtained from interrogation of arrays of the invention can be quantitative (e.g. measuring binding or catalytic constants $K_D$ & $K_M$), semiquantitative (e.g. normalising amount bound against protein quantity) or qualitative (e.g. functional vs. non-functional). By quantifying the signals for replicate arrays where the ligand is added at several (for example, two or more) concentrations, both the binding affinities and the active concentrations of protein in the spot can be determined. For example, quantitative results, $K_D$ and $B_{max}$, which describe the affinity of the interaction between ligand and protein and the number of binding sites for that ligand respectively, can be derived from protein array data. Briefly, either quantified or relative amounts of ligand bound to each individual protein spot can be measured at different concentrations of ligand in the assay solution. Assuming a linear relationship between the amount of protein and bound ligand, the (relative) amount of ligand bound to each spot over a range of ligand concentrations used in the assay can be fitted to equation 1, rearrangements or derivations.

$$\text{Bound ligand} = B_{max} / ((K_D/[\text{L}])+1) \qquad\qquad \text{(Equation 1)}$$

[L] = concentration of ligand used in the assay

The inventors have produced a functional array of membrane protein accessory proteins to provide a tool for the rapid investigation of binding parameters and specificity of ligands. The ligands can be other proteins, such as binding domains of other membrane protein subunits, peptides or potential therapeutic compounds to modulate complex assembly. The methodology provides for the determination of accurate binding constants in a highly parallel format.
The invention will now be further described by way of the following example which refers to the following figures in which:

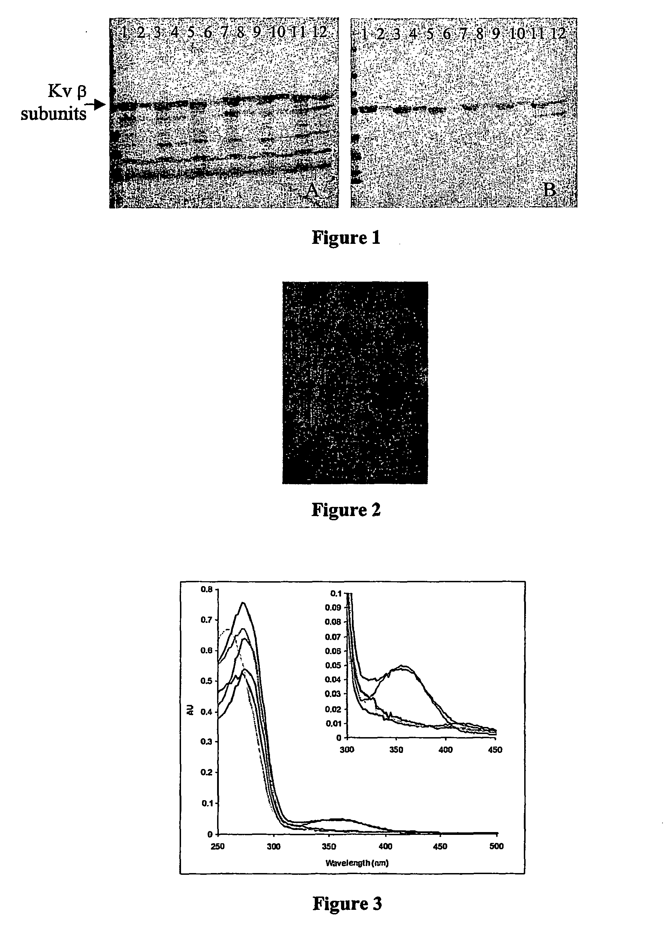

Figure 1 shows western blots of β subunit crude lysates. 10 μl of crude lysate and cleared lysate from each of the β subunit expressing clones were ran using SDS PAGE. Gels were transferred and membranes were probed with streptavidin (A) and anti His antibody (B). Lanes were loaded: 1&2 Human β1, 3&4 Human β2, 5&6 Human β3, 7&8 Rat β1, 9&10 Rat β2, 11&12 Rat β3. Odd lanes -crude lysate, even lanes - cleared lysate.

Figure 2 shows SDS PAGE of purified Kv β subunits. Lanes 1&8 Mr markers, 2 Human β1, 3 Human β2,4 Human β3, 5 Rat β1, 6 Rat β2, 7 Rat β3

Figure 3 shows UV-Visible absorbance spectra of purified Human and Rat Kv β subunits. Legend: Blue; Human β1, pink; Human β2, yellow; Human β3, cyan; Rat β1, purple; Rat β2, brown; Rat β3. Inset shows in more detail the peak at 360 nm.

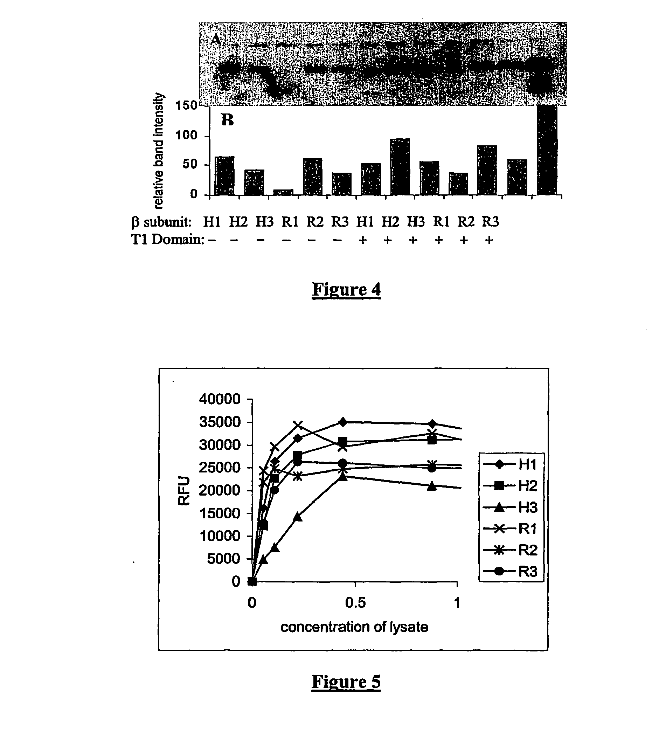

Figure 4 shows a Kv β subunit co-precipitation assay. A; Kv β subunit lysates were added to Kv α subunit coated anti FLAG beads (lanes 7-12) and to uncoated anti FLAG beads (lanes 1-6). After incubation for 1h the beads were washed, and boiled in SDS sample buffer before running on SDS PAGE. The gel was transferred to nitrocellulose membrane and biotinylated Kv β subunits visualized by using a streptavidin HRP conjugate. B; the bands from the western blot were quantified and plotted.

Figure 5 shows immobilisation of Kvβ subunits in streptavidin coated microtitre plates. Kvβ subunit lysates (original concentration approximately 3.5 mg/ml) were serially diluted and added to wells of a streptavidin coated microtitre plate. After washing to remove unbound protein, Kvβ subunits were quantified by using a Cy3 labelled anti His antibody. Bound antibody was quantified fluorometrically in a Packard fusion microtitre plate reader equipped with 550 and 570 nm excitation and emission filters respectively.

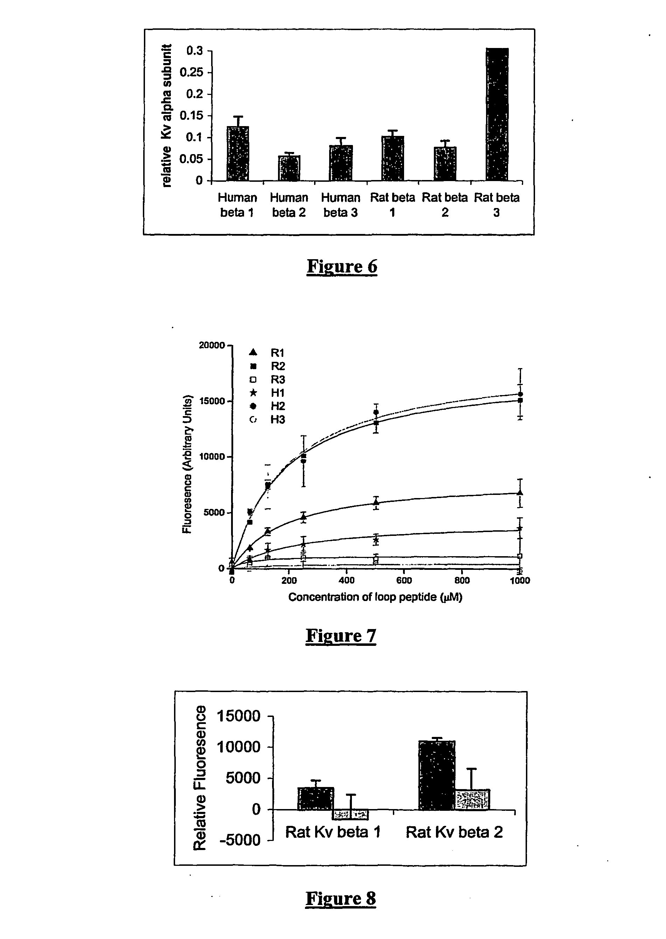

Figure 6 shows binding of Kv α subunit T1 domain to the Kvβ subunit microtitre plate array. Diluted crude α subunit T1 domain lysate was added to each well of a Kvβ subunit microtitre plate array. After incubation the wells were washed to remove unbound Kv α subunit T1 domain. Remaining bound Kv α subunit T1 domain was measured by using the FLAG tag and anti FLAG antibodies and visualized by using a HRP conjugate secondary antibody and colourmetric substrate.

Figure 7 shows binding curves for SPL2 peptide to Kv β subunit array. Peptide SPL2 was bound to the Kv β subunit array. After washing the amount remaining bound to the array was quantified fluorimetrically. The data has been fitted to binding curves. Blue triangles; Rat Kv β1, Black squares; Rat Kv β2, open pink squares; Rat Kv β3, red stars; Human Kv β1, green circles; Human Kv β2, Open cyan circles; Human Kv β3.

Figure 8 shows inhibition of peptide binding to Kyβ subunit array by Kv α subunit T1 domain. SPL2 peptide at approximately $K_d$ was added to wells of Kyβ subunit array in the presence and absence of purified Kv α subunit T1 domain. After washing, bound SPL2 peptide was quantified fluorimetrically

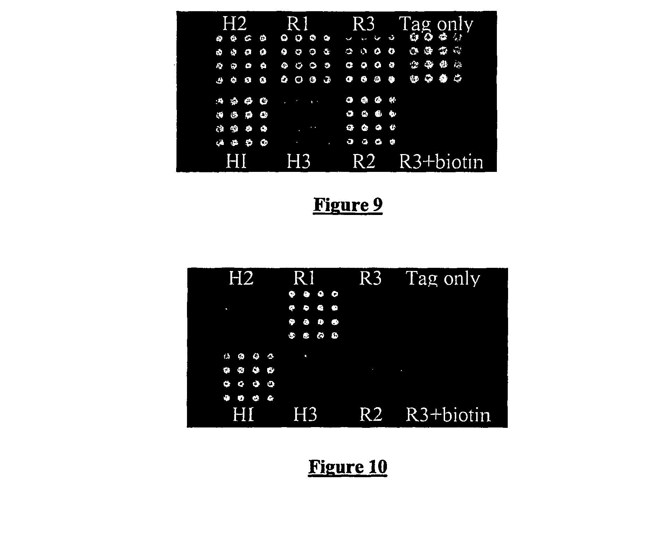

Figure 9 shows an antibody detection of microarrayed Kv β subunits. All Kv β subunits could be detected by using Cy3 labelled anti His antibody.

Figure 10 shows Kv α subunit T1 domain bound to microarrayed Kyβ subunits. A Kyβ subunit microarray was incubated with Kv α subunit T1 domain lysate. After washing Kv α subunit T1 domain bound to the β subunits could be detected using an anti FLAG antibody and Cy3 labelled anti mouse IgG.

## Example 1

Voltage gated potassium ($K_v$) channels are recognised as therapeutic targets in many disorders including those of the CNS, heart, lungs and bladder. They are therefore important therapeutic and commercial targets. They consist of a tetrameric assembly of α-subunits which have six transmembrane spanning domains and are coupled to an assembly of cytoplasmic regulatory β-subunits.

To date, molecular cloning has identified at least 23 different gene products encoding Kv channel α subunits. Further diversification resulting from alternative splicing has also been demonstrated for a number of α subunits. Four families of channel-forming Kv subunits, Kv1 to Kv4, are derived from the *Drosophila* potassium channels, *Shaker, Shab, Shaw* and *Shal,* respectively. Each of these channel types has multiple subfamily members. A further four families of Kv channels, Kv5, Kv6, Kv8 and Kv9, have been identified that do not form functional channels alone. They can, however, combine with the channel-forming subunits to form functional channels with altered biophysical properties. At least two of these families of auxiliary α subunits (Kv6 and Kv9) have multiple subfamily members.

In addition to the molecular diversity afforded by homomeric and heteromeric tetramerisation of Kv channel α subunits, cytoplasmic β subunits can bind to the channel complex and modulate channel kinetics or cell surface expression. Three families, Kvβ1 to Kvβ3, have been identified to date, with 3 splice variants observed from the Kvβ1 gene. The β-subunit complex binds to a region of the N-terminus of the α-subunit known as the T1 domain and the presence of the subunit increases cell-surface expression of the functional ion channel. An additional role for Kvβ1 subunits in modulating channel gating kinetics has also been established.

*Kv β subunit array*

**Materials and Methods for construction of Kv β subunit array**

*Cloning of His and biotin tagged Kyβ subunit cDNAs*

The Rat and Human Kv β1,β2 and β3 subunit core domains cDNA were ligated into an *E.coli* expression vector downstream of sequence coding for a poly Histidine-tag and the BCCP domain from the *E.coli AccB* gene. The ligation mix was transformed into chemically competent XL10-Gold cells (Stratagene) according to the manufacturer's instructions. The Kyβ subunit cDNA sequence was checked by sequencing and found to correspond to the expected protein sequence - see Appendix.

*Expression of Kyβ subunits in E.coli*

Colonies of XL10-Gold cells containing Kyβ subunit plasmids were inoculated into 5 ml of LB medium containing ampicillin in 20 ml tubes and grown overnight at 37°C in a shaking incubator. 2 ml of overnight culture was used to inoculate another 200 ml of LB/ampicillin in 500 ml flasks and grown at 37°C until an OD600 of~1.0 was reached. IPTG and biotin was then added to final concentrations of 1mM and 50μM respectively and induction continued at 23°C for 4 hours. Cells were then harvested by centrifugation, cell pellets were washed in PBS x 3 and stored in aliquots at-80°C.

*Lysis of E. coli containing Kyβ subunits*

Cell pellets were thawed on ice and 400 μl of lysis buffer (PBS containing 0.1 % Tween 20, 1 mg/ml lysozyme and 1μg/ml DNAse I) was added and the cells were resuspended by pipetting. Lysis was aided by incubation on a rocker at room temperature for 30min before cell debris was collected by centrifugation at 13000rpm for 10min at 4°C. The cleared supernatant of soluble protein was removed and used immediately.

*Purification of Kyβ subunits*

Kyβ subunits were purified by use of the hexahistidine tag. Cleared lysates diluted in PBS containing 50 mM imidazole were added to a column containing Talon Cobalt affinity resin (Clontech). The column was washed with 10 column volumes of buffer and protein was eluted in 2 column volumes of PBS containing 300 mM imidazole.

**SDS PAGE**

Protein samples were boiled in SDS containing buffer for 5min prior to loading on 4-20% Tris-Glycine gels (NOVEX) and run at 200V for 45min. Gels were then used for Western blotting or protein was stained directly with Coomassie brilliant blue dye.

**Western blotting**

Protein was transferred onto PVDF membrane (Hybond-P, Amersham) and probed for the presence of various epitopes using standard techniques. For detection of the histidine-tag, membranes were blocked in 5% Marvel/PBST and anti-RGSHis antibody (QIAGEN) was used as the primary antibody at 1/1000 dilution. For detection of the biotin tag, membranes were blocked in Superblock/TBS (Pierce) and probed with Streptavidin/HRP conjugate (Amersham) at 1/2000 dilution in Superblock/TBS/0.1% Tween20. The secondary antibody for the RGSHis antibody was anti-mouse IgG (Fc specific) HRP conjugate (Sigma) used at 1/2000 dilution in Marvel/PBST. After extensive washing, bound HRP conjugates were detected using either ECLPlus (Amersham) and Hyperfilm ECL (Amersham) or by DAB staining (Pierce).

**Spectra**

UV-Vis Spectra (250-500 nm) of purified Kyβ subunits were recorded using a Thermo spectronic scanning spectrophotometer. Proteins were diluted in elution buffer and elution buffer alone was used to take the baseline which is subtracted from all samples.

*α subunit T1 domain interaction assay*

The cDNA for the α subunit T1 domain (amino acids 33 - 135) was cloned downstream of sequences coding for a His-tag and a FLAG-tag in an *E.coli* expression vector. Plasmids were checked by sequencing for correct sequence and induction of *E.coli* cultures showed expression of a His and FLAG tagged soluble protein of the expected size. To test for interaction between α subunit T1 domain and Kyβ subunits, binding reactions were assembled containing 10μl Kyβ subunit cleared lysates, 10μl anti-FLAG agarose in the presence and absence of 10μl α subunit T1 domain cleared lysate, in 500μl phosphate buffered saline containing 300mM NaCl, 0.1 % Tween20 and 1% (w/v) bovine serum albumin. Reactions were incubated on a rocker at room temperature for 1 hour and FLAG bound complexes harvested by centrifugation at 5000rpm for 2min. After extensive washing in PBST, FLAG bound complexes were denatured in SDS sample buffer and Western blotted. Presence of biotinylated Kyβ subunits were detected by Streptavidin/HRP conjugate.

*Kyβ subunit microtitre plate array fabrication*

To 96 well streptavidin coated microtitre plates 100 μl of optimised concentrations of Kyβ subunit cleared lysates (typically 1 in 16 dilutions in PBS-Tween) were added to each well. Plates were incubated at room temperature with shaking for 45 mins before washing each well 3 times with 300 μl of PBS-Tween. After which the plates were used in assays or stored at - 20°C in the presence of 50% (v/v) glycerol in PBS-Tween.

*Kyβ subunit array peptide binding assay*

Kyβ subunit interacting peptide (SPL2) based on a portion of the α subunit domain was designed, synthesized and fluorescein labeled. SPL2 peptide appropriately diluted in PBS-Tween containing 0.1% w/v BSA was added at 100 μl/well to the Kyβ subunit coated microtitre plate. The peptide was incubated for 1 h at roomtemperature with shaking before unbound peptide was removed by washing 3 timed in 300 μl of PBS-Tween. 100 μl of 6M Guanidine HC1 was added to each well before evaluation of Fluorescein labelled SPL2 peptide concentration in each well. The fluorescence of each well of a 96 well microtitre plates were read in a Packard fusion microtitre plate reader equipped with 485 and 520 nm excitation and emission filters respectively.

*Kvβ subunit array Kv α subunit T1 domain interaction assay*

Kv α subunit T1 domain cleared lysate was diluted 1 in 10 in PBS-Tween containing 0.1% w/v BSA was added at 100 μl/well to the Kvβ subunit coated microtitre plate. The peptide was incubated for 1 h at room temperature with shaking before unbound α subunit T1 domain was removed by washing 3 timed in 300 μl of PBS-Tween. Bound α subunit T1 domain was estimated by using appropriately diluted mouse anti FLAG antibody and anti mouse IgG secondary antibody HRP conjugate using standard ELISA procedures. HRP was detected by addition of colourmetric HRP substrate2,2'-azino-bis(3-ethylbenzthiazoline-6-sulfonic acid) and microtitre plates were read in a Packard fusion microtitre plate reader equipped with a 405 nm absorbance filter.

*Kvβ subunit microarray fabrication*

Cleared lysates of Kvβ subunit were loaded into a 384 well plate and printed onto glass microscope slides with a neutravidin immobilized on dextran coating (Xantec) using a Qarray microarraying robot (Genetix, UK) with a 4 pin microarraying head. Each lysate was spotted 16 times onto each array. After printing, arrays were incubated in a humid chamber at 4°C for 1 h before washing in PBS-Tween. After washing arrays were ready for assay.

*Antibody Detection of microarrayed Kvβ subunits*

Cy3 labelled anti His antibody was diluted to 1 μg/ml in PBS-Tween + 0.1% (w/v) BSA and approximately 500 μl was pipetted onto the slide. The slide was incubated at room temperature with very slight shaking for 1 h before washing 2 x 2 mins in a large volume of PBS-Tween. Slides were centrifuged briefly at 2000g to remove liquid before scanning in a microarray scanner (Affymetrix 428 array scanner) equipped with excitation laser and emission filters appropriate for Cy3 fluorophore detection.

*Kv α subunit T1 domain Binding to microarrayed Kvβ subunits*

Kv α subunit T1 domain cleared lysate was diluted 1 in 50 in PBS-Tween containing 1% w/v BSA and approximately 500 μl was pipetted onto the slide. The slide was incubated at room temperature with very slight shaking for 1 h before washing 2 x 2 mins in a large volume of PBS-Tween. Appropriately diluted Anti FLAG Antibody was then pipetted onto the array and incubated and washed as above before addition of Cy3 labelled anti mouse IgG. The slides were incubated and washed as above before centrifuging briefly at 2000g to remove liquid before scanning in a microarray scanner (Affymetrix 428 array scanner) equipped with excitation laser and emission filters appropriate for Cy3 fluorophore detection.

*Results*

*Expression of Kvβ subunits in E.coli*

The Kvβ subunit core domains were cloned downstream of the tac promoter in vector pQE80 into which the BCCP domain from the *E.coli* gene ACCB had already been cloned. The resultant protein would then be His and biotin tagged at its N-terminus. Figure 1 shows Western blot analysis of total and soluble protein from induced E.coli cultures. For all β subunits there are clear bands for His-tagged and biotinylated protein with a molecular weight of approximately 50 kDa. Bands of the same size are detected by a polyclonal antibody raised to human Kv β2 (Biosource) which cross-reacted with all the β subunits used (Data not shown).

Although His and biotin tags have been used in this array, other affinity tags (e.g. FLAG, myc, VSV) could be used to enable purification, detection and immobilization of the cloned proteins. Also an expression host other than *E. coli* may be used (e.g. yeast, insect cells, mammalian cells) if required.

*Purification of Kvβ subunits*

Proteins can be purified before arraying by using an affinity tag. Here we have made use of the his tag by purifying the protein on Talon resin. Figure 2 shows that Kv β subunits purified in this way show only bands on SDS-PAGE that correspond to the Kv β subunit, or proteolysis products.

*UV- Vis Absorbance Spectra*

UV-Vis spectra were acquired for all Human and Rat Kv β subunits. Figure 3 shows that both Human and Rat Kv β2 subunits contain a NAD(P)H cofactor as evidenced by their absorbance peaks at 360 nm.

*α subunit T1 domain interaction assay*

To confirm that the expressed Kv β subunits have retained native function an assay was devised where the Kv β subunits would be precipitated by binding to α subunit T1 domain coated beads. To anti FLAG beads FLAG tagged Kv α subunit T1 domain lysates were added. These were then mixed with lysates containing the Kv β subunits. Figure 4 shows that although the non-specific binding of Kv β subunits to anti FLAG beads was high, binding in the presence of the Kv α subunit T1 domain was increased. Also it can be seen that Rat Kv β3 subunit has bound to Kv α subunit T1 domain coated beads in greater amounts than any other Kv β subunit.

*Kyβ subunit microtitre plate array fabrication*

To confirm that streptavidin coated microtitre plates can be coated with Kv β subunits, lysates were serially diluted and added to wells of plates. Figure 5 shows that similar levels of specifically immobilized protein per well can be achieved.

*Kyβ subunit array α subunit T1 domain interaction assay*

Figure 6 demonstrates specific binding of Kv α subunit T1 domain to all Kyβ subunits. It can also be noted that Rat Kv β3 subunit has bound more Kv a subunit T1 domain by far, mirroring the results of the co-precipitation assay.

*Kyβ subunit array peptide binding assay*

SPL2 peptide was added to a Kyβ subunit microtitre plate array at a range of concentrations from 100 to 1600 μM. After incubation for 1h the wells were washed to remove unbound peptide. The remaining peptide was quantifies fluorometrically. The results (shown in Fig 7) provide fits to the data allowing estimations of $K_d$ and $B_{max}$ for each of the Kyβ subunits (Table 1).

Table 1. Estimates of $K_d$ and $B_{max}$ for each of the Kyβ subunits. The data in Figure 7 were fitted to binding curves using curve-fitting software.

| Beta Subunit | Kd (μM) |
|---|---|
| R1 | 189.6 |
| R2 | 196.6 |
| R3 | 54.6 |
| H1 | 225.8 |
| H2 | 184.1 |
| H3 | 116.4 |

*Inhibition of peptide binding to Kyβ subunit array by Kv α subunit T1 domain*

Binding of SPL2 peptide to Kyβ subunit array was inhibited in the presence of Kv α subunit T1 domain. In Figure 8 it can be seen that for Rat Kyβ1 and 2 subunits peptide binding is reduced.

*Antibody Detection of microarrayed Kyβ subunits*

Microarrayed Kyβ subunits are shown to be specifically immobilised on streptavidin coated glass microscope slides. In Figure 9 it can be seen that the inclusion of free biotin in the spotting solution completely inhibited the immobilisation of Kv β3. All Kv β subunits are present on the array also the immobilisation BCCP tag is present on the array.

*Kv α subunit T1 domain Binding to microarrayed Kyβ subunits*

Kv β subunits immobilised on an array are still capable of binding Kv α subunit T 1 domain. Figure 10. shows that Kv α subunit T 1 domain can be detected after binding to immobilised Kv β subunits. It can also be seen from Figure 10 that the immobilised affinity tag has little binding of the Kv α subunit T1 domain.

**References**

1. Jan LY and Jan YN (1997) Receptor-regulated ion channels. *Curr Opin Cell Biol* 9(2): 155-60

2. Trimmer, J.S. (1998) Regulation of ion channel expression by cytoplasmic subunits. Curr. Opin. Neurobiol. 8, 370-374.

3. Perez-Garcia, *MT et al.,* (1995) Functional properties of cardiac L-type calcium channels transiently expressed in HEK293 cells. Roles of alpha 1 and beta subunits. *J. Gen. Physiol.* 105, 289-305.

4. Brady AE, Limbird LE. (2002) G protein-coupled receptor interacting proteins: Emerging roles in localization and signal transduction . *Cell Signal* 14(4):297-309

5. Ruiz-Velasco V, Ikeda SR, Puhl HL. (2002) Cloning, tissue distribution, and functional expression of the human G protein {beta}4-subunit. *Physiol Genomics* 8(1):41-50

6. Walker, D *et al.* (1998) A $\beta_4$ isoform-specific interaction site in the carboxyl-terminal region of the voltage-dependent $Ca^{2+}$ channel $\alpha_{1A}$ subunit. *J. Biol. Chem.,* 273, 2361-2367.

7. Xu and Li, (1998) Auxiliary Subunits of Shaker-type potassium channels. *Trends. Cardiovasc. Med.,* 8, 229-234.

8. Zhu H, Klemic JF, Chang S, Bertone P, Casamayor A, Klemic KG, Smith D, Gerstein M, Reed MA, Snyder M. Analysis of yeast protein kinases using protein chips. *Nat Genet* (2000) 26(3):283-9

9. Dolly, JO and Parcej, DN (1996) Molecular properties of voltage-gated K+ channels. *J. Bioenerg. Biomembr.,* 28, 231-253.

10. Scott, VE *et al.,* (1990) Alpha-dendrotoxin acceptor from bovine brain is a K+ channel protein. Evidence from the N-terminal sequence of its larger subunit. *J. Biol. Chem.,* 265, 20094-20097.

11. Wang, Z *et al.,* (1996) Comparison of Binding and Block Produced by Alternatively Spliced Kvβ1 Subunits. *J. Biol. Chem.,* 271, 28311-28317.

12. Restituito, S *et al.,* (2001) $Ca^{2+}$ Channel Inactivation Heterogeneity Reveals Physiological Unbinding of Auxiliary Subunits. *Biophys. J.* **81**, 89-96.

13. Strader, CD *et al.* (1994) Structure and function of G-protein-coupled receptors. *Annu. Rev. Biochem.,* **63**, 101-132.

14. Strader, CD *et al.* (1995) The family of G-protein-coupled receptors. *FASEB J.,* **9**, 745-754.

15. Tucker, S.J. and Ashcroft, F.M. (1999) Mapping of the physical interaction between the intracellular domains of an inwardly rectifying potassium channel, Kir6.2. *J. Biol. Chem.,* **274**, 33393-33397.

16. Yokoyama, CT *et al.* (1997) Phosphorylation of the synaptic protein interaction site on N-type calcium channels inhibits interactions with SNARE proteins. *J. Neurosci.,* **17**, 6929-6938.

17. Gelber, E *et al.,* (1999) Structure and function of the third intracellular loop of the 5-HT$_{2A}$ receptor: The third intracellular loop is α-helical and binds purified arrestins.

18. Weixel, KM and Bradbury, NA (2001) $\mu$2 Binding directs the cystic fibrosis transmembrane conductance regulator to the clathrin-mediated endocytic pathway. *J. Biol. Chem.,* **49**, 46251-46259.

19. Tu, JC *et al.* (1998) Homer binds a novel proline-rich motif and links group 1 metabotropic glutamate receptors with IP3 receptors. *Neuron, 21*, 717-726.

20. Shieh, B-H and Zhu, M-Y (1996) Regulation of the TRP $Ca^+$ channel by INAD in Drosophila photoreceptors. *Neuron, 16*, 991-998.

21. Pongs, O., Leicher, T., Berger, M. *et al.* (1999) Functional and molecular aspects of voltage-gated $K^+$ channel $\beta$ subunits.

22. Walker, D. and Waard, M: (1998) Subunit interaction sites in voltage-dependent Ca2$^+$ channels: role in channel function.

23. Onrust, R, Herzmark, P *et al.* (1997) Receptor and $\beta\gamma$ binding sites in the $\alpha$ subunit of the retinal G-protein transcducin. *Science, 275*, 381-384

24. Bourne, HR (1997) How receptors talk to trimeric G proteins. *Curr. Opin. Cell. Biol.,* 9, 134-142.

25. Ferguson, SSG (2001) Evolving concepts in G protein-coupled receptor endocytosis: The role in receptor desensitization and signaling. *Pharmacol. Rev.,* 53, 1-24.

26. Krupnick, JG *et al.,* (1994) Arrestin-rhodopsin interaction. Multi-site binding delineated by peptide inhibition. *J. BioL Chem.* 269, 3226-3232.

27. Nam, *T-W et al.,* (2001) The *Escherichia coli* glucose transporter enzyme IICB$^{Glc}$ recruits the global repressor Mlc. *EMBO J.,* 20, 491-498.

28. Zhang, *J-Z et al.,* (2001) Overexpression of stomatin depresses GLUT-1 glucose transporter activity. *Am. J. Physiol. Cell. Physiol.,* 280, C1277-C1283.

**Appendix 1**

*Protein Sequence Human Kv $\beta$1 subunit core domain (residues 87- 419):*

```
GTGMKYRNLGKSGLRVSCLGLGTWVTFGGQISDEVAERLMTIAYESG
VNLFDTAEVYAAGKAEVILGSIIKKKGWRRSSLVITTKLYWGGKAETE
RGLSRKHIIEGLKGSLQRLQLEYVDVVFANRPDSNTPMEEIVRAMTHVI
NQGMAMYWGTSRWSAMEIMEAYSVARQFNMIPPVCEQAEYHLFQRE
KVEVQLPELYHKIGVGAMTWSPLACGIISGKYGNGVPESSRASLKCYQ
WLKERIVSEEGRKQQNKLKDLSPIAERLGCTLPQLAVAWCLRNEGVSS
VLLGSSTPEQLIENLGAIQVLPKMTSHVVNEIDNILRNKPYSKKDYRS
```

*Protein Sequence Human Kv $\beta$2 subunit core domain (residues 36 - 364):*

GLQFYRNLGKSGLRVSCLGLGTWVTFGGQITDEMAEQLMTLAYDNGI
NLFDTAEVYAAGKAEVVLGNIKKKGWRRSSLVITTKIFWGGKAETER
GLSRKHIIEGLKASLERLQLEYVDVVFANRPDPNTPMEETVRAMTHVIN
QGMAMYWGTSRWSSMEIMEAYSVARQFNLTPPICEQAEYHMFQREK
VEVQLPELFHKIGVGAMTWSPLACGIVSGKYDSGIPPYSRASLKGYQW
LKDKILSEEGRRQQAKLKELQAIAERLGCTLPQLAIAWCLRNEGVSSVL
LGASNADQLMENIGAIQVLPKLSSSIIHEIDSILGNKPYSKKDYRS

*Protein Sequence Human Kv β3 subunit core domain (residues 75 - 404):*

GTGMKYRNLGKSGLRVSCLGLGTWVTFGSQISDETAEDVLTVAYEHG
VNLFDTAEVYAAGKAERTLGNILKSKGWRRSSYVITTKIFWGGQAETE
RGLSRKHIIEGLRGSLERLQLGYVDIVFANRSDPNCPMEEIVRAMTYVI
NQGLALYWGTSRWGAAEIMEAYSMARQFNLIPPVCEQAEHHLFQREK
VEMQLPELYHKIGVGSVTWYPLACGLITSKYDGRVPDTCRASIKGYQW
LKDKVQSEDGKKQQAKVMDLLPVAHQLGCTVAQLAIAWCLRSEGVSS
VLLGVSSAEQLIEHLGALQVLSQLTPQTVMEIDGLLGNKPHSKK

*Protein Sequence Rat Kv β1 subunit core domain:*

GTGMKYRNLGKSGLRVSCLGLGTWVTFGGQISDEVAERLMTIAYESG
VNLFDTAEVYAAGKAEVILGSIKKKGWRRSSLVITTKLYWGGKAETE
RGLSRKHIIEGLKGSLQRLQLEYVDVVFANRPDSNTPMEEIVRAMTHVI
NQGMAMYWGTSRWSAMEIMEAYSVARQFNMIPPVCEQAEYHLFQRE
KVEVQLPELYHKIGVGAMTWSPLACGIISGKYGNGVPESSRASLKCYQ
WLKERIVSEEGRKQQNKLKDLSPIAERLGCTLPQLAVAWCLRNEGVSS
VLLGSSTPEQLIENLGAIQVLPKMTSHVVNEIDNILRNKPYSKKDYRS

*Protein Sequence Rat Kv β2 subunit core domain:*

GLQFYRNLGKSGLRVSCLGLGTWVTFGGQITDEMAEHLMTLAYDNGI
NLFDTAEVYAAGKAEVVLGNIKKKGWRRSSLVITTKIFWGGKAETER
GLSRKHIIEGLKASLERLQLEYVDVVFANRPDPNTPMEETVRAMTHVIN
QGMAMYWGTSRWSSMEIMEAYSVARQFNLIPPICEQAEYHMFQREKV

EVQLPELFHKIGVGAMTWSPLACGIVSGKYDSGIPPYSRASLKGYQWL
KDKILSEEGRRQQAKLKELQAIAERLGCTLPQLAIAWCLRNEGVSSVLL
GASNAEQLMENIGAIQVLPKLSSSIVHEIDSILGNKPYSKKDYRS .

*Protein Sequence Rat Kv β3 subunit core domain (residues 75 - 404):*

GTGMKYRNLGKSGLRVSCLGLGTWVTFGSQISDETAEDLLTVAYEHG
VNLFDTAEVYAAGKAERTLGNILKSKGWRRSSYVITTKIFWGGQAETE
RGLSRKHIIEGLQGSLDRLQLEYVDIVFANRSDPSSPMEEIVRAMTYVIN
QGLALYWGTSRWSAAEIMEAYSMARQFNLIPPVCEQAENHFFQREKV
EMQLPELYHKIGVGSVTWSPLACSLITSKYDGQVPDACKATVKGYQW
LKEKVQSEDGKKQQARVTDLLPIAHQLGCTVAQLAIAWCLRSEGVSSV
LLGVSSAEQLMEHLGSLQVLGQLTPQTVMEIDALLGNKSHSKK

**Claims**

1. An array comprising a surface having attached thereto at least one cytosolic accessory protein of a membrane protein selected from ion channels, G protein coupled receptors and transmembrane transporter proteins, wherein said cytosolic accessory protein is free from membrane protein components or other subunits of said ion channel, G protein coupled receptor or transmembrane transporter protein complex.

2. An array as claimed in claim 1, comprising a plurality of cytosolic accessory proteins selected from ion-channel subunits, G protein coupled receptor cytosolic accessory proteins, transmembrane transporter cytosolic accessory proteins, $K^+$-channel β-subunits, $Ca^{2+}$-channel β-subunits, G protein subtypes, Kv channel β-subunits, Calcium channel β-subunits, Gs family, Gt family, Gi family, Gi-0 family, Gq-11 family, Gα-sensory family and βγ family proteins.

3. An array as claimed in claim 1, comprising a plurality of cytosolic accessory proteins which are identical and are selected from ion-channel subunits, G protein coupled receptor cytosolic accessory proteins, transmembrane transporter cytosolic accessory proteins, $K^+$-channel β-subunits, $Ca^{2+}$-channel β-subunits, G protein subtypes, Kv channel β-subunits, Calcium channel β-subunits, Gs family, Gt family, Gi family, Gi-0 family, Gq-1 1 family, Gα-sensory family and βγ family proteins.

4. An array as claimed in claim 2 or 3, wherein the array comprises at least one $K^+$-channel β-subunit selected from: β1.1, β1.2, β1.3, β2.1, β2.2, β3.1, β3.2 and β4.

5. An array as claimed in claim 2 or 3, wherein the array comprises at least one calcium channel β-subunit selected from: β1a, β1b, β1c, β2a, β2b, β2c, β3a, β3b and β4.

6. An array as claimed in any preceding claim, wherein the cytosolic accessory protein is an ion channel subunit domain.

7. An array as claimed in any preceding claim, wherein cytosolic accessory protein subunits are provided as tagged protein constructs.

8. An array as claimed in claim 7, wherein the tagged protein construct comprises an affinity tag.

9. An array as claimed in claim 7 or claim 8, wherein the tag is a His, biotin, FLAG, myc, or VSV tag.

10. An array as claimed in any preceding claim, wherein the protein moieties are attached to the surface via a common marker moiety.

11. An array as claimed in any preceding claim, wherein each position in the array contains one or more copies of a single protein type in the form of a monomer, dimer, trimer, tetramer or higher multimer.

12. A method for determining which cytosolic proteins interact with a given membrane protein, or vice versa, said method comprising the steps of :

(i) providing an array of cytosolic accessory proteins as claimed in any preceding claim;
(ii) contacting the array with cytosolic fragments of said membrane protein and/or cytosolic fragments of other related membrane protein family members; and
(iii) detecting and identifying the interacting partners.

13. A method for screening compounds or peptides or proteins for the ability to interact selectively with a cytosolic protein, said method comprising the steps of :

(i) providing an array as claimed in any of claims 1-11;
(ii) contacting the array with candidate compounds or peptides or proteins; and
(iii) identifying the interacting partners.

14. A method as claimed in claim 13, further comprising the step of quantitating the interaction of the interacting partners by measuring the binding or catalytic constants $K_D$ or $K_M$, or by normalising amount bound against protein quantity.

15. A method for screening compounds or peptides or proteins for the ability to selectively modulate the interaction between a cytosolic protein and a membrane protein, said method comprising the steps of:

(i) providing an array as claimed in any of claims 1-11;
(ii) contacting the array with candidate compounds or peptides or proteins and with one or more membrane proteins or cytosolic fragments thereof, either simultaneously or in sequence; and
(iii) determining whether said interaction is modulated by the presence of said compounds or peptides or proteins.

16. A method according to claim 15 wherein the cytosolic fragment of a membrane protein is a soluble functional domain.

17. A method as claimed in claim 15 or claim 16, further comprising the step of quantitating the degree of modulation of the interaction by measuring the binding or catalytic constants $K_D$ or $K_M$, or by normalising amount bound against protein quantity.

18. A method as claimed in any one of claims 15 to 16 wherein the concentration of a compound required to inhibit a given interaction is measured by determination of the $IC_{50}$ value.

19. The use of an array of cytosolic accessory proteins as claimed in any of claims 1-11 to measure the relative catalytic activity of different members of a family of said cytosolic accessory proteins.

20. The use of an array of cytosolic accessory proteins as claimed in any of claims 1-11 as an affinity surface on which to select antibodies from a library of phenotype-genotype-linked antibodies (such as phage displayed antibodies).

21. The use of an array of cytosolic accessory proteins as claimed in any of claims 1-11 for determining the effect of post-translational modifications on the interactions of said cytosolic accessory proteins with membrane proteins.

**Patentansprüche**

1. Anordnung umfassend eine Oberfläche, die darauf angeheftet wenigstens ein zytosolisches akzessorisches Protein von einem Membranprotein aufweist, ausgewählt aus Ionenkanälen, G-Protein gekoppelten Rezeptoren und transmembranen Transporterproteinen, wobei das zytosolische akzessorische Protein frei von Membranproteinbestandteilen oder anderen Untereinheiten des Ionenkanals, G-Protein gekoppelten Rezeptors oder transmembranen Transporterproteinkomplexes ist.

2. Anordnung wie beansprucht in Anspruch 1, umfassend eine Vielzahl von zytosolischen akzessorischen Proteinen ausgewählt aus Ionenkanal Untereinheiten, G-Protein gekoppelten Rezeptor- zytosolischen akzessorischen Proteinen, transmembranen Transporter- zytosolischen akzessorischen Proteinen, $K^+$-Kanal β-Untereinheiten, $Ca^{2+}$-Kanal β-Untereinheiten, G-Protein Subtypen, Kv Kanal β-Untereinheiten, Calciumkanal β-Untereinheiten, Gs Familien-, Gt Familien-, Gi Familien-, Gi-0 Familien-, Gq-11 Familien-, Gα-sensorischen Familien- und βγ Familienproteinen.

3. Anordnung wie beansprucht in Anspruch 1, umfassend eine Vielzahl von zytosolischen akzessorischen Proteinen, die identisch sind und ausgewählt sind aus Ionenkanal Untereinheiten, G-Protein gekoppelten Rezeptor-, zytosolischen akzessorischen Proteinen, transmembranen Transporter-, zytosolischen akzessorischen Proteinen, $K^+$-Kanal β-Untereinheiten, $Ca^{2+}$-Kanal β-Untereinheiten, G-Protein Subtypen, Kv Kanal β-Untereinheiten, Calciumkanal β-Untereinheiten, Gs Familien-, Gt Familien-, Gi Familien-, Gi-0 Familien-, Gq-11 Familien-, Ga-sensorischen Familien- und βγ Familienproteinen.

4. Anordnung wie beansprucht in Anspruch 2 oder 3, wobei die Anordnung wenigstens eine $K^+$-Kanal β-Untereinheit umfasst ausgewählt aus: β1.1, β1.2, β1.3, β2.1, β2.2, β3.1, β3.2 und β4.

5. Anordnung wie beansprucht in Anspruch 2 oder 3, wobei die Anordnung wenigstens eine Calciumkanal β-Untereinheit umfasst ausgewählt aus: β1a, β1b, β1c, β2a, β2b, β2c, β3a, β3b und β4.

6. Anordnung wie beansprucht in einem vorhergehenden Anspruch, wobei das zytosolische akzessorische Protein eine Ionenkanal Untereinheitsdomäne ist.

7. Anordnung wie beansprucht in einem vorhergehenden Anspruch, wobei zytosolische akzessorische Proteinuntereinheiten als markierte Proteinkonstrukte bereitgestellt werden.

8. Anordnung wie beansprucht in Anspruch 7, wobei das markierte Proteinkonstrukt einen Affinitätsmarker umfasst.

9. Anordnung wie beansprucht in Anspruch 7 oder Anspruch 8, wobei der Marker ein His, Biotin, FLAG, myc oder VSV Marker ist.

10. Anordnung wie beansprucht in einem vorhergehenden Anspruch, wobei die Proteinreste an die Oberfläche über einen allgemeinen bzw. gemeinsamen Markerrest angeheftet sind.

11. Anordnung wie beansprucht in einem vorhergehenden Anspruch, wobei jede Position in der Anordnung eine oder mehrere Kopien einess einzelnen Proteintyp in der Form eines Monomers, Dimers, Trimers, Tetramers oder höheren Multimers enthält.

12. Verfahren zum Bestimmen, welche zytosolischen Proteine mit einem gegebenen Membranprotein, oder vice versa, interagieren, das Verfahren umfassend die Schritte des:

    (i) Bereitstellens einer Anordnung von zytosolischen akzessorischen Proteinen wie beansprucht in einem vorangegangenen Anspruch;
    (ii) In-Kontaktbringens der Anordnung mit zytosolischen Fragmenten des Membranproteins und/oder der zytosolischen Fragmente von anderen verwandten Membranprotein Familienmitgliedern; und
    (iii) Nachweisens und Identifizierens der Interagierenden Partner.

13. Verfahren zum Screenen von Verbindungen oder Peptiden oder Proteinen nach der Fähigkeit, selektiv mit einem zytosolischen Protein zu interagieren, das Verfahren umfassend die Schritte des:

    (i) Bereitstellens einer Anordnung wie beansprucht in einem der Ansprüche 1-11;
    (ii) In-Kontaktbringens der Anordnung mit Kandidatenverbindungen oder Peptiden oder Proteinen; und
    (iii) Identifizierens der interagierenden Partner.

14. Verfahren wie beansprucht in Anspruch 13, ferner umfassend den Schritt des Quantifizierens der Interaktion der interagierenden Partner durch Messen der Bindungs- oder katalytischen Konstanten $K_D$ oder $K_M$ oder durch Normalisieren von gebundener Menge gegenüber Proteinquantität.

**15.** Verfahren zum Screenen von Verbindungen oder Peptiden oder Proteinen nach der Fähigkeit, selektiv die Interaktion zwischen einem zytosolischen Protein und einem Membranprotein zu modulieren, das Verfahren umfassend die Schritte des:

(i) Bereitstellens einer Anordnung wie beansprucht in einem der Ansprüche 1-11;
(ii) In-Kontaktbringens des Arrays mit Kandidatenverbindungen oder Peptiden oder Proteinen und mit einem oder mehreren Membranproteinen oder zytosolischen Fragmenten davon, entweder gleichzeitig oder nacheinander; und
(iii) Bestimmens, ob die Interaktion durch die Anwesenheit der Verbindungen oder Peptide oder Proteine moduliert wird.

**16.** Verfahren gemäß Anspruch 15, wobei das zytosolische Fragment eines Membranproteins eine lösliche, funktionelle Domäne ist.

**17.** Verfahren wie beansprucht in Anspruch 15 oder Anspruch 16, ferner umfassend den Schritt des Quantifizierens des Grades der Modulation der Interaktion durch Messen der Bindungs- oder katalytischen Konstanten $K_D$ oder $K_M$ oder durch Normalisieren von gebundener Menge gegenüber Proteinquantität.

**18.** Verfahren wie beansprucht in einem der Ansprüche 15 bis 16, wobei die Konzentration einer Verbindung, die benötigt wird, um eine gegebene Interaktion zu inhibieren, durch Bestimmung des $IC_{50}$ Werts gemessen wird.

**19.** Verwendung einer Anordnung von zytosolischen akzessorischen Proteinen wie beansprucht in einem der Ansprüche 1-11, um die relative katalytische Aktivität von verschiedenen Mitgliedern einer Familie der zytosolischen akzessorischen Proteine, zu messen.

**20.** Verwendung einer Anordnung von zytosolischen akzessorischen Proteinen wie beansprucht in einem der Ansprüche 1-11 als eine Affnitätsoberfläche, um Antikörper aus einer Bibliothek von Phänotyp-Genotyp-verknüpften Antikörpern (wie durch Phagen Display entwickelte Antikörper) auszuwählen.

**21.** Verwendung einer Anordnung von zytosolischen akzessorischen Proteinen wie beansprucht in einem der Ansprüche 1-11 zum Bestimmen des Effekts von posttranslationalen Modifikationen auf die Interaktionen der zytosolischen akzessorischen Proteine mit Membranproteinen.

**Revendications**

**1.** Réseau comprenant une surface à laquelle est attachée au moins une protéine accessoire cytosolique d'une protéine de membrane choisie parmi les canaux ioniques, les récepteurs couplés à la protéine G et les protéines transporteuses transmembranaires, dans lequel ladite protéine accessoire cytosolique est exempte de composants de protéine de membrane ou d'autres sous-unités dudit canal ionique, récepteur couplé à la protéine G ou complexe de protéines transporteuses transmembranaires.

**2.** Réseau selon la revendication 1, comprenant une pluralité de protéines accessoires cytosoliques choisies parmi des sous-unités de canaux ioniques, des protéines accessoires cytosoliques de récepteurs couplés à la protéine G, des protéines accessoires cytosoliques transporteuses transmembranaires, des sous-unités $\beta$ de canaux K$^+$, des sous-unités $\beta$ de canaux Ca$^{2+}$, des sous-types de protéine G, des sous-unités $\beta$ de canaux Kv, des sous-unités $\beta$ de canaux calciques, et des protéines de la famille Gs, la famille Gt, la famille Gi, la famille Gi-0, la famille Gq-11, la famille sensorielle G$\alpha$ et la famille $\beta\gamma$.

**3.** Réseau selon la revendication 1, comprenant une pluralité de protéines accessoires cytosoliques qui sont identiques et sont choisies parmi des sous-unités de canaux ioniques, des protéines accessoires cytosoliques de récepteurs couplés à la protéine G, des protéines accessoires cytosoliques transporteuses transmembranaires, des sous-unités $\beta$ de canaux K$^+$, des sous-unités $\beta$ de canaux Ca$^{2+}$, des sous-types de protéine G, des sous-unités $\beta$ de canaux Kv, des sous-unités $\beta$ de canaux calciques, et des protéines de la famille Gs, la famille Gt, la famille Gi, la famille Gi-0, la famille Gq-11, la famille sensorielle G$\alpha$ et la famille $\beta\gamma$.

**4.** Réseau selon la revendication 2 ou 3, lequel réseau comprend au moins une sous-unité $\beta$ de canaux K$^+$ choisie parmi : $\beta$1.1, $\beta$1.2, $\beta$1.3, $\beta$2.1, $\beta$2.2, $\beta$3.1, $\beta$3.2 et $\beta$4.

**5.** Réseau selon la revendication 2 ou 3, lequel réseau comprend au moins une sous-unité β de canaux calciques choisie parmi : β1a, β1b, β1c, β2a, β2b, β2c, β3a, β3b et β4.

**6.** Réseau selon l'une quelconque des revendications précédentes, dans lequel la protéine accessoire cytosolique est un domaine de sous-unité de canal ionique.

**7.** Réseau selon l'une quelconque des revendications précédentes, dans lequel les sous-unités de protéines accessoires cytosoliques se présentent sous 1a forme de produits d'assemblage protéiques étiquetés.

**8.** Réseau selon la revendication 7, dans lequel le produit d'assemblage protéique étiqueté comprend une étiquette d'affinité.

**9.** Réseau selon la revendication 7 ou la revendication 8, dans lequel l'étiquette est une étiquette His, biotine, FLAG, myc, ou VSV.

**10.** Réseau selon l'une quelconque des revendications précédentes, dans lequel les fragments protéiques sont attachés à la surface via un fragment marqueur commun.

**11.** Réseau selon l'une quelconque des revendications précédentes, dans lequel chaque position dans le réseau contient une ou plusieurs copies d'un type de protéine unique sous la forme d'un monomère, d'un dimère, d'un trimère, d'un tétramère ou d'un multimère supérieur.

**12.** Procédé pour déterminer quelles protéines cytosoliques interagissent avec une protéine de membrane donnée, ou vice versa, ledit procédé comprenant les étapes consistant à :

(i) se procurer un réseau de protéines accessoires cytosoliques selon l'une quelconque des revendications précédentes ;
(ii) amener le réseau en contact avec des fragments cytosoliques de ladite protéine de membrane et/ou des fragments cytosoliques d'autres membres d'une famille de protéines de membrane apparentée ; et
(iii) détecter et identifier les partenaires qui interagissent.

**13.** Procédé pour sélectionner des composés ou peptides ou protéines quant à leur aptitude à interagir sélectivement avec une protéine cytosolique, ledit procédé comprenant les étapes consistant à :

(i) se procurer un réseau selon l'une quelconque des revendications 1 à 11 ;
(ii) amener le réseau en contact avec des composés ou peptides ou protéines candidats ; et
(iii) identifier les partenaires qui interagissent.

**14.** Procédé selon la revendication 13, comprenant en outre l'étape consistant à quantifier l'interaction des partenaires qui interagissent en mesurant les constantes de liaison ou catalytique $K_D$ ou $K_M$, ou en normalisant la quantité liée en fonction par rapport à la quantité de protéine.

**15.** Procédé pour sélectionner des composés ou peptides ou protéines quant à leur aptitude à moduler sélectivement l'interaction entre une protéine cytosolique et une protéine de membrane, ledit procédé comprenant les étapes consistant à :

(i) se procurer un réseau selon l'une quelconque des revendications 1 à 11 ;
(ii) amener le réseau en contact avec des composés ou peptides ou protéines candidats et avec un(e) ou plusieurs protéines de membrane ou fragments cytosoliques de celles-ci, soit simultanément soit successivement ; et
(iii) déterminer si ladite interaction est modulée par la présence desdits composés ou peptides ou protéines.

**16.** Procédé selon la revendication 15, dans lequel le fragment cytosolique d'une protéine de membrane est un domaine fonctionnel soluble.

**17.** Procédé selon la revendication 15 ou la revendication 16, comprenant en outre l'étape consistant à quantifier le degré de modulation de l'interaction en mesurant les constantes de liaison ou catalytique $K_D$ ou $K_M$, ou en normalisant la quantité liée par rapport à la quantité de protéine.

**18.** Procédé selon l'une quelconque des revendications 15 et 16, dans lequel la concentration d'un composé requise pour inhiber une interaction donnée est mesurée par détermination de la valeur $CI_{50}$.

**19.** Utilisation d'un réseau de protéines accessoires cytosoliques selon l'une quelconque des revendications 1 à 11 pour mesurer l'activité catalytique relative de différents membres d'une famille desdites protéines accessoires cytosoliques.

**20.** Utilisation d'un réseau de protéines accessoires cytosoliques selon l'une quelconque des revendications 1 à 11 en tant que surface d'affinité sur laquelle on sélectionne des anticorps à partir d'une bibliothèque d'anticorps liés à un phénotype-génotype (tels que des anticorps présentés sur phage).

**21.** Utilisation d'un réseau de protéines accessoires cytosoliques selon l'une quelconque des revendications 1 à 11 pour déterminer l'effet de modifications post-traductionnelles sur les interactions desdites protéines accessoires cytosoliques avec des protéines de membrane.

Kv β
subunits →

**Figure 1**

**Figure 2**

**Figure 3**

β subunit:  H1  H2  H3  R1  R2  R3  H1  H2  H3  R1  R2  R3
T1 Domain:  –   –   –   –   –   –   +   +   +   +   +   +

**Figure 4**

**Figure 5**

**Figure 6**

**Figure 7**

**Figure 8**

**Figure 9**

**Figure 10**